(19) Europäisches Patentamt
European Patent Office
Office européen des brevets

(11) EP 3 185 926 B1

(12) **EUROPÄISCHE PATENTSCHRIFT**

(45) Veröffentlichungstag und Bekanntmachung des Hinweises auf die Patenterteilung:
**28.11.2018 Patentblatt 2018/48**

(21) Anmeldenummer: **15763840.4**

(22) Anmeldetag: **25.08.2015**

(51) Int Cl.:
***A61M 1/34*** $^{(2006.01)}$ ***A61M 1/36*** $^{(2006.01)}$
***B01D 63/02*** $^{(2006.01)}$ ***B01D 69/02*** $^{(2006.01)}$

(86) Internationale Anmeldenummer:
**PCT/EP2015/069415**

(87) Internationale Veröffentlichungsnummer:
**WO 2016/030357 (03.03.2016 Gazette 2016/09)**

(54) **SYSTEM ZUR ENTFERNUNG VON PROINFLAMMATORISCHEN MEDIATOREN SOWIE VON GRANULOZYTEN UND MONOZYTEN AUS BLUT**

SYSTEM FOR THE REMOVAL OF PRO-INFLAMMATORY MEDIATORS AND GRANULOCYTES AND MONOCYTES FROM BLOOD

SYSTÈME D'ÉLIMINATION DE MÉDIATEURS PRO-INFLAMMATOIRES ET GRANULOCYTES ET MONOCYTES SANGUINS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.08.2014 EP 14182261**

(43) Veröffentlichungstag der Anmeldung:
**05.07.2017 Patentblatt 2017/27**

(73) Patentinhaber: **3M Innovative Properties Company**
**St. Paul, MN 55133-3427 (US)**

(72) Erfinder:
- **VON HARTEN, Bodo**
  **42349 Wuppertal (DE)**
- **KRIETER, Detlef**
  **97074 Würzburg (DE)**
- **LEMKE, Horst Dieter**
  **63785 Obernburg (DE)**

(74) Vertreter: **Hettstedt, Stephan et al**
**3M Deutschland GmbH**
**3M Office of Intellectual Property Counsel**
**Carl-Schurz Str. 1**
**41453 Neuss (DE)**

(56) Entgegenhaltungen:
**EP-A1- 2 380 610** **WO-A2-2008/083965**
**DE-U1- 20 321 776** **US-A1- 2013 161 247**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 185 926 B1

**Beschreibung**

Die vorliegende Erfindung betrifft ein System zur Behandlung von Blut, insbesondere zur Behandlung von Sepsis.

Sepsis und systemische Entzündungsreaktionen (SIRS) sind mit Mortalitätsraten zwischen 30-70% die häufigste Todesursache auf Intensivstationen. Bei einer Sepsis handelt es sich um eine Erkrankung, die durch eine komplexe systemische Entzündungsreaktion des Organismus auf das Eindringen infektiöser Erreger gekennzeichnet ist. Die Entzündungsreaktion führt zu Organdysfunktionen unterschiedlichen Ausmaßes und endet häufig mit dem Tod des Patienten. Betroffene Patienten versterben dabei weniger an den direkten Auswirkungen der bakteriellen Infektion, sondern vor allem an den systemischen Auswirkungen der oftmals überschießenden inflammatorischen Reaktion des Körpers. Die Kontrolle dieser Immunantwort, bei der die Produktion sogenannter proinflammatorischer Mediatoren wie z.B. Zytokinen durch neutrophile Granulozyten und Monozyten eine Schlüsselrolle einnimmt, ist mit Fortschreiten der Sepsis zunehmend schwerer beherrschbar.

In der Standardtherapie der Sepsis sind die Gabe von Antibiotika und supportive Maßnahmen, d.h. bedarfsadaptiert die Gabe von kreislaufstützenden Medikamenten, künstliche Beatmung und Nierenersatztherapie, etabliert. Die zusätzliche Gabe zytokinhemmender Substanzen hat sich hingegen als nicht wirksam erwiesen. In den vergangenen Jahren wurden darüber hinaus verschiedene Konzepte zur Entfernung von Zytokinen vorgestellt, zum einen durch extrakorporale Hemofiltration, zum anderen durch extrakorporale Adsorption.

Konzepte zur Entfernung von Zytokinen oder allgemein proinflammatorischer Mediatoren durch extrakorporale Hemofiltration werden beispielsweise in der US-A 5 571 418, der US2012/0312732, der EP-A 2 281 625, der WO 03/009885 oder der WO 2011/131534 beschrieben. Die extrakorporale Adsorption von Zytokinen ist beispielsweise Gegenstand der DE-A 199 13 707, der WO 2013/025483, der WO2012/094565 oder der US 2013/0011824.

Schließlich sind Verfahren und Vorrichtungen zu deren Durchführung beschrieben, bei denen Zytokine über Membranfilter in Gestalt z.B. von Ultrafiltern oder Plasmafiltern mit dem Permeat vom Blut abgetrennt werden und bei denen anschließend das die Zytokine enthaltene Permeat durch einen Adsorber zur spezifischen Adsorption der Zytokine hindurchgeleitet und schließlich das so gereinigte Permeat dem Patienten zurückgeführt wird (siehe z.B. WO 00/02603, WO03/009885, US2012/0312732, EP-A 0 787 500, EP-A 0 958 839). Das Dokument US2013/0161247 A1 enthüllt einen Plasmafilter und eine Kolonne, um Zytokine in Serie in einer Blutkreislaufschleife zu eliminieren.

Versuche am septischen Tiermodell wiesen dabei auf einen positiven Effekt der Zytokinfiltration (am Schwein) bzw. Zytokinadsorption (an der Ratte) hin. Erste Studien am Menschen konnten dies jedoch bisher nicht bestätigen, obwohl eine effektive Reduktion der Zytokinkonzentrationen im Blut nachgewiesen werden konnte.

Nachteil der reinen Zytokinentfernung durch extrakorporale Verfahren - ob durch Filtration oder Adsorption - ist, dass inflammatorisch aktivierte, zytokingenerierende Leukozyten hierdurch nicht eliminiert werden und deren fortbestehende überschießende Immunantwort und somit der Krankheitsprozess weiter unterhalten werden.

Die WO 2007/025735 beschäftigt sich mit der Behandlung von Sepsis und verfolgt den Ansatz, dass aktivierte Leukozyten zur Produktion von Zytokinen beitragen, die ihrerseits wiederum weitere Immunzellen aktivieren, was schließlich zur systemischen Überreaktion des Immunabwehrsystems und zur Sepsis führt. Zur Behandlung der Sepsis schlägt die WO 2007/025735 daher einen Filter zur Entfernung von aktivierten Leukozyten vor, bei dem Blut im sogenannten Dead-End Modus durch ein Filtermaterial strömt, in dem die Leukozyten zurückgehalten und so aus dem Blut entfernt werden. Das Filtermaterial kann in einer Ausführungsform mit Liganden oder anderen bioaktiven Substanzen versehen sein, die beispielsweise spezifisch mit Zytokinen wechselwirken und eine zusätzliche Entfernung von Zytokinen bei der Durchströmung des zu behandelnden Bluts durch das Filtermaterial bewirken.

Die vorliegende Erfindung hat zur Aufgabe, ein System zur Behandlung von Blut zur Verfügung zu stellen, mittels dessen insbesondere an Sepsis erkrankte Patienten in effizienter Weise behandelt werden können.

Die Aufgabe wird durch ein Blutbehandlungssystem gelöst, umfassend mindestens eine erste Vorrichtung und mindestens eine zweite Vorrichtung,

- wobei die mindestens eine erste Vorrichtung einen ersten blutseitigen Strömungspfad zum Hindurchleiten von Blut und die mindestens eine zweite Vorrichtung einen zweiten blutseitigen Strömungspfad zum Hindurchleiten von Blut aufweist und wobei die mindestens eine erste Vorrichtung und die mindestens eine zweite Vorrichtung so seriell hintereinandergeschaltet sind, dass der erste blutseitige Strömungspfad in Fluidverbindung mit dem zweiten blutseitigen Strömungspfad steht,
- wobei die mindestens eine erste Vorrichtung ein Membranfilter zur Entfernung von toxischen Mediatoren aus Blut ist und die mindestens eine zweite Vorrichtung zur Entfernung von Granulozyten und Monozyten aus Blut geeignet ist und
- wobei der Membranfilter ein Gehäuse, einen im Gehäuse ausgebildeten Filterinnenraum und eine im Filterinnenraum angeordnete semipermeable Membran aufweist, die den Filterinnenraum in einen Retentatraum und einen Permeatraum unterteilt,

- wobei das Gehäuse eine Bluteinlasseinrichtung und eine Blutauslasseinrichtung aufweist, die mit dem Retentatraum in Fluidverbindung stehen, und die Bluteinlasseinrichtung, der Retentatraum und die Blutauslasseinrichtung den ersten blutseitigen Strömungspfad zum Hindurchleiten von Blut durch die erste Vorrichtung ausbilden und
- wobei das Gehäuse des Weiteren einen Permeatauslass zum Ableiten von durch die semipermeable Membran hindurchtretendem Permeat aus dem Permeatraum aufweist und
- wobei die semipermeable Membran eine Ultrafiltrationsrate in Wasser UFRwasser im Bereich von 500 bis 2000 ml/(h m2 mmHg) und der Membranfilter eine Trenncharakteristik derart aufweist, dass der Siebkoeffizient für Albumin SKAlb im Bereich von 0,05 bis 0,3 und der Siebkoeffizient für Immunglobulin G (IgG), SKIgG, im Bereich von 0,001 bis 0,1 liegt.

In der Anwendung erfolgt mit dem erfindungsgemäßen Blutbehandlungssystem eine kaskadenförmige, d.h. aufeinanderfolgende Behandlung des das System durchströmenden und zu behandelnden Bluts, bei der in der mindestens einen ersten Vorrichtung proinflammatorische Mediatoren, wie z.B. Zytokine, und in der zweiten Vorrichtung Granulozyten und Monozyten entfernt werden. Dabei kann das Blut das erfindungsgemäße System so durchlaufen, dass es zunächst durch die mindestens eine erste Vorrichtung hindurchströmt und damit zuerst die proinflammatorischen Mediatoren entfernt werden und anschließend die mindestens eine zweite Vorrichtung zur Entfernung der Granulozyten und Monozyten. Ebenso ist es möglich, dass das Blut das erfindungsgemäße System so durchläuft, dass es zunächst durch die mindestens eine zweite Vorrichtung hindurchströmt, wobei dann damit zuerst die Granulozyten und Monozyten entfernt werden, und anschließend die mindestens eine erste Vorrichtung zur Entfernung der proinflammatorischen Mediatoren.

Die mindestens eine erste und mindestens eine zweite Vorrichtung sind so miteinander verbunden, dass der erste blutseitige Strömungspfad zum Hindurchleiten von Blut der ersten Vorrichtung in Fluidverbindung mit dem zweiten blutseitigen Strömungspfad der zweiten Vorrichtung steht. Daraus ergibt sich, dass das in das System eingeleitete Blut beim Durchlaufen der mindestens einen ersten und der mindestens einen zweiten Vorrichtung in zwei voneinander unabhängigen Schritten nacheinander behandelt wird. Die Vorgänge der Entfernung von proinflammatorischen Mediatoren und der Entfernung von Granulozyten und Monozyten sind voneinander entkoppelt und finden jeweils am Blut selbst statt. Die Entkopplung hat den Vorteil, dass die einzelnen Vorrichtungen spezifisch auf den jeweiligen Entfernungsprozess ausgerichtet werden können.

Je nach Anwendungsfall ist es möglich, dass das System zur Blutbehandlung eine einzelne erste Vorrichtung und eine einzelne zweite Vorrichtung umfasst, deren blutseitige Strömungspfade miteinander verbunden sind. Es ist jedoch auch möglich, dass z.B. eine einzelne erste Vorrichtung mit zwei zueinander parallel geschalteten zweiten Vorrichtungen verbunden ist, so dass das die erste Vorrichtung verlassende und bzgl. proinflammatorischer Mediatoren gereinigte Retentat auf zwei Stoffströme aufgeteilt und den zweiten blutseitigen Strömungspfaden der beiden zweiten Vorrichtungen zugeführt wird. Ebenso ist es möglich, dass das System zur Blutbehandlung eine einzelne erste Vorrichtung und eine einzelne zweite Vorrichtung umfasst und das die erste Vorrichtung verlassende Retentat auf zwei Stoffströme aufgeteilt wird, wobei der eine Stoffstrom die einzelne zweite Vorrichtung durchströmt und der zweite Stoffstrom im Bypass ohne eine weiteren Behandlung geführt wird. Der die zweite Vorrichtung verlassende und bzgl. Granulozyten und Monozyten gereinigte Stoffstrom sowie der im Bypass geführte Stoffstrom können dann in Strömungsrichtung gesehen hinter der zweiten Vorrichtung zusammengeführt werden und als Gesamtstrom z.B. dem zu behandelnden Patienten zugeführt werden. Natürlich sind weitere Kombinationen aus einzelnen oder mehreren ersten und zweiten Vorrichtungen im erfindungsgemäßen System zur Blutbehandlung möglich.

Im Membranfilter strömt das zu behandelnde Blut auf dem ersten blutseitigen Strömungspfad über die Bluteinlasseinrichtung in den Filterinnenraum und auf der Retentatseite der semipermeablen Membran durch den Retentatraum. Bei der Durchströmung des Retentatraums tritt ein Teil des zu behandelnden Bluts als Permeat bzw. Ultrafiltrat durch die semipermeable Membran hindurch, wobei die semipermeable Membran so hinsichtlich der Größe ihrer Poren ausgelegt ist, dass die aus dem Blut zu entfernenden proinflammatorischen Mediatoren als Teil des Permeats bzw. Ultrafiltrats durch die Poren in den Permeatraum transportiert werden können. Das so behandelte und hinsichtlich proinflammatorischer Mediatoren abgereicherte Blut verlässt auf dem ersten Strömungspad über die Blutauslasseinrichtung den Membranfilter, während das die aus dem Blut entfernten proinflammatorischen Mediatoren enthaltende Permeat den Membranfilter über den Permeatauslass verlässt.

In einer bevorzugten Ausführungsform ist die semipermeable Membran mindestens eine Hohlfasermembran mit einer Wand und einem von der Wand umschlossenen Lumen. Die mindestens eine Hohlfasermembran kann in einer bevorzugten Ausführungsform über ihre Wand eine asymmetrische Porenstruktur mit einer Trennschicht auf der dem Lumen zugewandten Seite der Wand der Hohlfasermembran aufweisen. Besonders bevorzugt ist eine Vielzahl von Hohlfasermembranen im Membranfilter zu einem Bündel angeordnet. In einer besonders bevorzugten Ausführungsform wird der Retentatraum des Membranfilters vom Lumen der mindestens einen Hohlfasermembran ausgebildet.

Die semipermeable Membran der Membranfilters ist vorzugsweise eine hydrophile Membran. Dabei ist in einer besonders bevorzugten Ausführungsform die hydrophile Membran aus einem hydrophoben ersten Polymer aufgebaut,

das mit einem hydrophilen zweiten Polymer kombiniert ist. Als hydrophobes erstes Polymere kommen technische Kunststoffe aus der Gruppe der aromatischen Sulfonpolymere, wie z.B. Polysulfon, Polyethersulfon, Polyphenylensulfon oder Polyarylethersulfon, der Polycarbonate, Polyimide, Polyetherimide, Polyetherketone, Polyphenylensulfide, Copolymere oder Modifikationen dieser Polymere oder Mischungen dieser Polymere in Frage. In einer besonders bevorzugten Ausführungsform ist das hydrophobe erste Polymer ein Polysulfon oder ein Polyethersulfon mit den in nachstehenden Formeln (I) und (II) dargestellten wiederkehrenden Moleküleinheiten

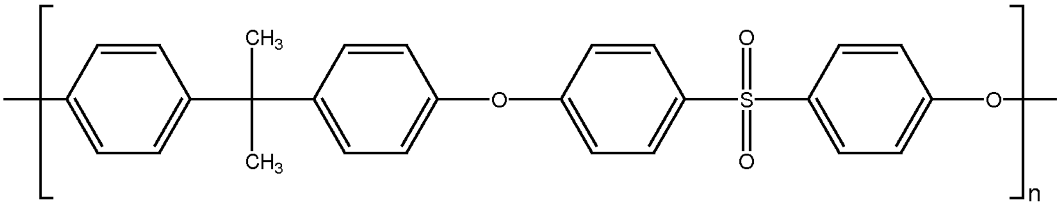

( I )

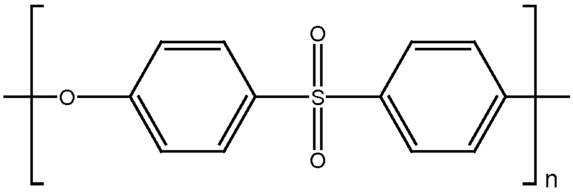

( II )

Als hydrophiles zweites Polymer werden vorteilhafterweise langkettige Polymere eingesetzt, die auf der einen Seite eine Kompatibilität zu dem synthetischen ersten Polymer aufweisen, und die über wiederkehrende polymere Einheiten verfügen, die an sich hydrophil sind. Vorzugsweise ist das hydrophile zweite Polymer Polyvinylpyrrolidon, Polyethylenglykol, Polyvinylalkohol, Polyglykolmonoester, Polysorbitat, wie z.B. Polyoxyethylensorbitanmonooleat, Carboxylmethylcellulose oder eine Modifikation oder ein Copolymer dieser Polymere. Besonders bevorzugt ist Polyvinylpyrrolidon.

Der Membranfilter zur Entfernung proinflammatorischer Mediatoren kann beispielsweise die Gestalt üblicher Hemofilter haben, bei denen die Zuführung und Abführung des zu behandelnden Bluts über eine Bluteinlasseinrichtung bzw. eine Blutauslasseinrichtung in den Endkappen des Membranfilters erfolgt, die mit den den Retentatraum ausbildenden Lumina der im Membranfilter bündelförmig angeordneten Hohlfasermembranen in Fluidverbindung stehen. In den Außenraum um die Hohlfasermembranen, d.h. in den Permeat- oder Filtratraum, mündet in der Regel über die Wand des Gehäuses zumindest eine Auslasseinrichtung, d.h. der Permeatauslass, über die das Permeat, welches die aus dem Blut entfernten proinflammatorischen Mediatoren enthält, den Membranfilter verlässt. Hinsichtlich der enthaltenen semipermeablen Membran unterscheidet sich der erfindungsgemäße Membranfilter jedoch von den üblichen Hemofiltern, wie nachfolgend ausgeführt wird.

In der mindestens einen ersten Vorrichtung, d.h. im Membranfilter zur Entfernung von toxischen Mediatoren aus Blut wird in der Anwendung von dem auf der Retentatseite strömenden Blut ein Teil des Plasmawassers als Ultrafiltrat entnommen, wobei das Ultrafiltrat die im Blut enthaltenen toxischen Mediatoren enthält, die auf Grund ihrer Molekülgröße im sogenannten Mittelmolekülbereich auf Grund der Trenncharakteristik der Membran durch die semipermeable Membran hindurch treten können. Wesentliche Bestandteile des Bluts wie die zellulären Bestandteile, größere im Blutplasma gelöste Proteine, wie Albumin, Immunglobuline, HDL oder LDL, Antikörper oder Fribrinogene werden hingegen zum überwiegenden Teil oder nahezu vollständig von der im Membranfilter enthaltenen semipermeablen Membran zurückgehalten. Der diese semipermeable Membran enthaltende erfindungsgemäße Membranfilter weist erfindungsgemäß einen Siebkoeffizienten für Albumin in Blut $SK_{Alb}$ im Bereich von 0,015 bis 0,35 auf. Bevorzugt liegt für den Membranfilter der Siebkoeffizient für Albumin in Blut $SK_{Alb}$ im Bereich von 0,05 bis 0,3 und besonders bevorzugt im Bereich von 0,1 bis 0,25. Die semipermeable Membran des erfindungsgemäßen Membranfilters lässt damit Albumin, an das zu entfernende toxische Mediatoren z.T. gebunden sein können, zu gewissen Anteilen durch. Erfindungsgemäße Membranfilter mit derartigen Siebkoeffizienten weisen eine Trenngrenze im Bereich von 50.000 bis 150.000 Dalton auf.

In einer weiteren bevorzugten Ausführungsform wird im Membranfilter bzw. durch die semipermeable Membran des Membranfilters Immunglobulin G (IgG) mit einem Molekulargewicht von ca. 180.000 Dalton nahezu vollständig zurückgehalten. Bevorzugt weist der Membranfilter einen Siebkoeffizienten für IgG $SK_{IgG}$ im Bereich von 0,001 bis 0,1 auf. Besonders bevorzugt liegt der Siebkoeffizient für IgG $SK_{IgG}$ im Bereich von 0,003 bis 0,08.

Die im erfindungsgemäßen Membranfilter enthaltene semipermeable Membran bzw. der erfindungsgemäße Membranfilter unterscheidet sich damit von im Bereich der Blutreinigung vielfach eingesetzten Plasmafiltern, die eine

Trenngrenze oberhalb von ca. zwei Millionen Dalton aufweisen und bei denen über eine Abtrennung von Blutplasma eine nahezu vollständige Abtrennung der o.g., im Blutplasma gelösten Bestandteile von den Blutzellen erfolgt. Die in derartigen Plasmafiltern enthaltenen Plasmafiltrationsmembranen haben also eine weitaus offenere Struktur als die Membranen des erfindungsgemäßen Membranfilters. Diese offenere Struktur wirkt sich gleichzeitig in hohen Durchlässigkeiten der Plasmafiltrationsmembranen aus, resultierend in Ultrafiltrationsraten für Wasser, $UFR_{Wasser}$, von oberhalb ca. 15.000 ml/(h $m^2$ mmHg).

Auf der anderen Seite unterscheidet sich der erfindungsgemäße Membranfilter bzw. die darin enthaltene semipermeable Membran von Hemodialysatoren, Hemodiafiltern oder Hemofiltern bzw. den darin eingesetzten Membranen, deren Trenngrenze mit bis zu ca. 40.000 Dalton in Vollblut so ausgebildet ist, dass sie Albumin und Moleküle größer als Albumin zumindest nahezu zurückhalten und bei denen Siebkoeffizienten für Albumin in Blut $SK_{Alb}$ von kleiner 0,005 realisiert werden.

Vorzugsweise besitzt die semipermeable Membran des vorliegenden Membranfilters eine Ultrafiltrationsrate in Wasser bzw. hydraulische Permeabilität $UFR_{Wasser}$ im Bereich von 500 bis 2000 ml/(h $m^2$ mmHg). Besonders bevorzugt liegt die hydraulische Permeabilität im Bereich von 500 bis 1500 ml/(h $m^2$ mmHg). Bestens geeignet ist eine semipermeable Membran mit einer $UFR_{Wasser}$ im Bereich von 800 bis 1200 ml/(h $m^2$ mmHg). Hierdurch wird eine genügend schnelle Entfernung der toxischen Mediatoren während der Blutbehandlung erreicht..

In der Anwendung kann das im Membranfilter erzeugte Permeat bzw. Ultrafiltrat, das die aus dem Blut entfernten proinflammatorischen Mediatoren enthält, verworfen werden. Es ist jedoch auch möglich, das Permeat bzw. Ultrafiltrat aufzureinigen, d.h. die proinflammatorischen Mediatoren aus dem Permeat beispielsweise über geeignete Adsorber zu entfernen, und das gereinigte Permeat dem behandelten Blutstrom zurückzuführen.

Als zweite Vorrichtung, welche zur Entfernung von Granulozyten und Monozyten ausgelegt und geeignet ist, können beispielsweise Zentrifugalgeräte eingesetzt werden. Vorzugsweise handelt es sich jedoch bei der mindestens einen zweiten Vorrichtung zur Entfernung von Granulozyten und Monozyten um einen Filter, um einen Adsorber oder um eine Kombination aus beiden, die zur Entfernung von Granulozyten und Monozyten ausgelegt und geeignet sind.

In einer bevorzugten Ausführungsform kann die mindestens eine zweite Vorrichtung ein Filter zur Entfernung von Granulozyten und Monozyten sein mit einem Filtergehäuse, welches einen Innenraum sowie eine Einlasseinrichtung und eine Auslasseinrichtung aufweist, die mit dem Innenraum in Fluidverbindung stehen,

- wobei im Innenraum des Filtergehäuses ein Strömungskanäle aufweisendes und in diesen Strömungskanälen von Blut durchströmbares Filtermaterial angeordnet ist,
- wobei Einlasseinrichtung, Auslasseinrichtung und die Strömungskanäle des Filtermaterials im Innenraum den zweiten Strömungspfad ausbilden, und
- wobei das Filtermaterial für die Abtrennung von Granulozyten und Monozyten durch Größenausschluss und/oder durch Adsorption angepasst ist.

Bei dem Filtermaterial kann es sich um ein faseriges Material, beispielsweise um ein vliesartiges Material oder um ein Material in Gestalt einer oder mehrerer Gewebelagen handeln. Derartige Filter werden z.B. in der EP-A 0 155 003, der EP-A 1 444 996, der EP-A 1 553 113, der EP-A 1 582 228, der EP-A 1 754 496, der US 2011/0031191, der WO 2004/018078, der WO 2004/039474, der WO 2005/002647 oder der WO 2006/061862 beschrieben. In den in diesen Schriften offenbarten Filtern kann in den eingesetzten Filtermaterialien neben oder anstelle einer Entfernung von Granulozyten und Monozyten über Größenausschluss auch eine Entfernung über spezifische Wechselwirkungen zwischen dem Filtermaterial und den Granulozyten und Monozyten, d.h. über Adsorption erfolgen. Es sind auch Filter mit Filtermaterialien bekannt, deren Oberflächeneigenschaften für die Adsorption von Leukozyten, Granulozyten oder Monozyten angepasst sind (siehe z.B. EP-A 0 478 914, EP-A 0 606 646, EP-A 1 016 426, US-A 4 476 023, WO 2004/064980, WO 2008/028807). Solche Filter zur Entfernung von Leukozyten, Granulozyten und Monozyten sind auch unter der Handelsbezeichnung Cellsorba™ (Fa. Asahi Medical Co. Ltd.) kommerziell erhältlich.

Das Filtermaterial kann auch in Form poröser Materialien vorliegen, die von Blut, aus dem beispielsweise Leukozyten entfernt werden sollen, durchströmt werden können. Poröse Filtermaterialien in Gestalt semipermeabler Membranen werden z.B. in der EP-A 0 606 646, der EP-A 1 666 129 oder der US-A 5 478 470 offenbart.

In einer weiteren bevorzugten Ausführungsform des Systems zur Behandlung von Blut kann die mindestens eine zweite Vorrichtung zur Entfernung von Granulozyten und Monozyten aus Blut ein Adsorber mit einem Gehäuse sein, welches eine einen Innenraum umschließende Innenseite sowie eine Einlasseinrichtung und eine Auslasseinrichtung aufweist,

- wobei im Innenraum eine Vielzahl von Fäden angeordnet ist,
- wobei die Fäden mit zumindest einem ihrer Enden so in eine mit der Gehäuseinnenseite verbundenen Vergussmasse eingebettet sind, dass um die Fäden herum ein von Blut durchströmbarer Außenraum ausgebildet ist, der mit der Einlasseinrichtung und der Auslasseinrichtung in Fluidverbindung steht, wodurch Einlasseinrichtung, Auslasseinrichtung und Innenraum den zweiten Strömungspfad ausbilden,

- wobei die Anordnung der Fäden einen hohen Grad an Ordnung aufweist, wobei unter einem hohen Grad an Ordnung zu verstehen ist, dass ein Anteil von mindestens 25% der Fäden entlang ihrer Erstreckungsrichtung nebeneinander angeordnet sind und
- wobei die Fäden auf Basis organischer Polymere eine Bildung des Komplementaktivierungsproduktes C5a in einer Konzentration von mindestens 10 $\mu$g pro $m^2$ Fadenoberfläche bewirken.

Vorzugsweise handelt es sich bei den Fäden in diesen Vorrichtungen um Hohlfäden mit einem Lumen und einer das Lumen umschließenden Wand sowie mit einer inneren, lumenseitigen Oberfläche und einer äußeren Oberfläche, wobei die Hohlfäden im Gehäuse so angeordnet sind, dass nur die äußere Oberflächen der Hohlfäden für das den Außenraum durchströmende Blut zugänglich, die Lumina der Hohlfäden hingegen nicht für ein Fluid zugänglich sind.

Derartige Adsorber zur Entfernung von Leukozyten, wie z.B. Granulozyten und Monozyten, werden in der US 2008/0203024 und der US 2010/0084331 beschrieben, auf deren Offenbarung sich an dieser Stelle ausdrücklich bezogen wird.

In einer weiteren bevorzugten Ausführungsform kann die mindestens eine zweite Vorrichtung zur Entfernung von Granulozyten und Monozyten aus Blut ein Adsorber mit einem Gehäuse sein, welches eine einen Innenraum umschließende Innenseite sowie eine Einlasseinrichtung und eine Auslasseinrichtung aufweist, wobei im Innenraum ein aus Partikeln aufgebautes Adsorptionsmaterial angeordnet ist. Um die Partikel des Adsorptionsmaterials herum ist ein von Blut durchströmbarer Außenraum ausgebildet, der mit der Einlasseinrichtung und der Auslasseinrichtung in Fluidverbindung steht, wodurch Einlasseinrichtung, Auslasseinrichtung und Innenraum den zweiten Strömungspfad ausbilden. Das Blut strömt bei diesen Adsorbern in der Anwendung auf dem zweiten blutseitigen Strömungspfad über die Einlasseinrichtung in den Innenraum, umströmt die Adsorberpartikel und verlässt den Innenraum über die Auslasseinrichtung.

Vorzugsweise werden in den Adsorbern als Adsorberpartikel Partikel auf Basis von Celluloseacatat oder Styrol verwendet. Jedoch sind auch andere Materialien wie z.B. Polyamide, Polyethylenterephthalat oder Polyacylnitril bekannt, die einer Oberflächenmodifikation unterzogen wurden oder mit einer Beschichtung versehen sind.

Derartige Adsorber, wie sie als zweite Vorrichtung zur Entfernung von Granulozyten und Monozyten geeignet sind, sind auch Gegenstand verschiedener Patentpublikationen (s. z.B. EP-A 0 319 961, EP-A 1 882 738, WO 2000/55621, US-A 4 370 381) und sind als kommerzielle Produkte beispielsweise unter der Handelsbezeichnung Adacolumn® (Fa. JIMRO Co., Ld.) erhältlich.

In einer bevorzugten Ausführungsform des Systems zur Behandlung von Blut ist die mindestens eine erste Vorrichtung zur Entfernung von proinflammatorischen Mediatoren ein Membranfilter und die mindestens eine zweite Vorrichtung zur Entfernung von Granulozyten und Monozyten ein Adsorber mit einem Gehäuse, in dessen Innenraum eine Vielzahl von Fäden mit einem hohen Grad an Ordnung angeordnet sind, wie er zuvor beschrieben wurde. Besonders bevorzugt handelt es sich bei dem Membranfilter um einen solchen, bei dem die mindestens eine semipermeable Membran eine semipermeable Hohlfasermembran ist. Bei dieser bevorzugten Ausführungsform mit einem Membranfilter und einem Adsorber auf Basis von in einem hohen Grad an Ordnung angeordneten Fäden als erster und zweiter Vorrichtung sind die beiden Vorrichtungen so miteinander verbunden, dass der Retentatraum der ersten Vorrichtung mit dem die Fäden der zweiten Vorrichtung umgebenden Außenraum in Fluidverbindung steht. Bei der besonders bevorzugten Ausführungsform mit einem Membranfilter mit Hohlfasermembranen stehen die Lumina der semipermeablen Hohlfasern, die den Retentatraum darstellen, mit dem die Fäden der zweiten Vorrichtung umgebenden Außenraum in Fluidverbindung.

In der Anwendung strömt für diese Ausführungsformen das zu behandelnde Blut auf dem ersten blutseitigen Strömungspfad über die Bluteinlasseinrichtung des Membranfilters in den Retentatraum des Membranfilters ein, durchströmt diesen, wobei über Filtration ein Teil des zu behandelnden Bluts als Permeat durch die semipermeable Membran hindurchtritt und wobei bei der Filtration eine Entfernung von proinflammatorischen Mediatoren erfolgt. Nach Durchströmen des Retentatraums verlässt das Retentat den Membranfilter über die Blutauslasseinrichtung, während das die aus dem Blut entfernten proinflammatorischen Mediatoren enthaltende Ultrafiltrat über den Retentatauslass aus dem Membranfilter entfernt wird. Das Retentat, d.h. das in der ersten Vorrichtung behandelte Blut, strömt nach Verlassen des Membranfilters auf dem zweiten blutseitigen Strömungspfad über die Einlasseinrichtung des Adsorbers in den Außenraum um die im Adsorber angeordneten Fäden ein und überströmt die Fäden an ihrer Außenseite. Dabei erfolgt über Adsorption an den Fäden eine Entfernung von Granulozyten und Monozyten aus dem die Fäden überströmenden und zu behandelnden Blut.

Das dann auch in der zweiten Vorrichtung behandelte und nunmehr von proinflammatorischen Mediatoren sowie von Granulozyten und Monozyten gereinigte Blut verlässt dann den Adsorber über dessen Auslasseinrichtung.

Es ist ebenso möglich, dass das zu behandelnde Blut erst die erste Vorrichtung und dann die zweite Vorrichtung durchströmt. Für die obigen bevorzugten Ausführungsformen bedeutet dies, dass dann in der Anwendung das zu behandelnde Blut auf dem zweiten blutseitigen Strömungspfad über die Einlasseinrichtung des Adsorbers in den Außenraum um die im Adsorber angeordneten Fäden ein- und die Fäden an ihrer Außenseite überströmt. Dabei erfolgt über Adsorption an den Fäden eine Entfernung von Granulozyten und Monozyten aus dem die Fäden überströmenden und zu behandelnden Blut. Das in der zweiten Vorrichtung behandelte und von Granulozyten und Monozyten gereinigte Blut verlässt dann den Adsorber über dessen Auslasseinrichtung.

Dieses in der zweiten Vorrichtung behandelte Blut strömt nach Verlassen des Adsorbers auf dem ersten blutseitigen Strömungspfad über die Bluteinlasseinrichtung des Membranfilters in den Retentatraum des Membranfilters ein, durchströmt diesen, wobei über Filtration ein Teil des zu behandelnden Bluts als Permeat durch die semipermeable Membran hindurchtritt und bei der Filtration eine Entfernung von proinflammatorischen Mediatoren erfolgt. Nach Durchströmen des Retentatraums verlässt das Retentat, d.h. das auch in der ersten Vorrichtung behandelte Blut und nunmehr von proinflammatorischen Mediatoren sowie von Granulozyten und Monozyten gereinigte Blut den Membranfilter über dessen Blutauslasseinrichtung. Das die proinflammatorischen Mediatoren enthaltende Permeat wird über den Permeatauslass aus dem Membranfilter entfernt.

Mindestens eine erste Vorrichtung zur Entfernung proinflammatorischen Mediatoren und mindestens eine zweite Vorrichtung zur Entfernung von Granulozyten und Monozyten können als separate Vorrichtungen des erfindungsgemäßen Systems hintereinandergeschaltet werden, wobei, wie bereits ausgeführt wurde, die Reihenfolge der Vorrichtungen und die Reihenfolge, in der das zu behandelnde Blut die Vorrichtungen durchläuft, den Erfordernissen der Blutbehandlung angepasst werden können. Dabei müssen jedoch die Vorrichtungen so miteinander verbunden sein, dass der erste blutseitige Strömungspfad der mindestens einen ersten Vorrichtung und der zweite blutseitige Strömungspfad der mindestens einen zweiten Vorrichtung miteinander in Fluidverbindung stehen und die beiden blutseitigen Strömungspfade nacheinander von dem zu behandelnden Blut durchströmbar sind, d.h. in der Anwendung bei der Blutbehandlung nacheinander durchströmt werden. Erste Vorrichtung und zweite Vorrichtung können z.B. über geeignete Schlauchverbindungen, Anschlussstutzen oder Adapter miteinander verbunden sein.

Erste und zweite Vorrichtung können auch die Gestalt von Zylindern haben, die beispielsweise direkt über eine Klebe-, Schweiß-, Schraub- oder Flanschverbindung miteinander verbunden sind. Dabei kann die erste Vorrichtung ein Hohlfasermembranmodul sein, bei dem die Hohlfasermembranen in einem zylindrischen Gehäuse im wesentlichen zueinander parallel in Richtung der Längsachse des zylindrischen Gehäuses angeordnet und die Lumina der Hohlfasermembranen von den Gehäuseenden her anströmbar sind. Die zweite Vorrichtung mit ebenfalls einem zylindrischen Gehäuse, die z.B. eine Anordnung von Fäden zur Adsorption von Granulozyten und Monozyten, ein faseriges Filtermaterial oder ein partikelförmiges Adsorptionsmaterial enthalten kann, kann dann mit ihrem einen Gehäuseende an eines der Gehäuseenden der ersten Vorrichtung angeflanscht sein oder mit einem der Gehäuseenden der ersten Vorrichtung z.B. verklebt, verschweißt oder mittels einer Überwurfmutter verschraubt sein.

Auch andere integrale Bauformen des Systems zur Blutbehandlung sind möglich. Die zweite Vorrichtung kann als eine Endkappe ausgebildet sein, die eine mit einem Adsorbermaterial gefüllte Kammer aufweist und die auf eines der Enden einer zylindrisch ausgebildeten ersten Vorrichtung aufgeschraubt ist. Ebenso kann das System zur Behandlung von Blut so ausgebildet sein, dass die zweite Vorrichtung konzentrisch in Form eines Mantels um das Gehäuse einer ersten Vorrichtung mit zylinderförmiger Form angeordnet ist und so erste und zweite Vorrichtung eine integrale Einheit bilden.

Der Ermittlung der Kenngrößen zur Charakterisierung der semipermeablen Membran des Membranfilters der ersten Vorrichtung werden die folgenden Messmethoden zu Grunde gelegt:

Ermittlung der Siebkoeffizienten für den Membranfilter:

Die Siebkoefizienten SK werden für $\alpha_1$ saures Glykoprotein ($M_W$= 44.000 Dalton), $SK_{Gp}$, Albumin ($M_W$=68.000 Dalton), $SK_{Alb}$, und für Immunglobulin G ($M_W$=180.000 Dalton), $SK_{IgG}$, ermittelt. Die Bestimmung der Siebkoeffizienten wird gemäß DIN EN ISO 8637:2014-03, insbesondere Abschnitt 5.6.2 und Bild 5, mit frisch gespendetem humanen Heparinblut (10 IU/ml) an einer Dialysemaschine durchgeführt (Nikkiso DBB-03), wobei das Vollblut während der Messung rezirkulierend gefahren wird. Das Blut wird vor dem Experiment auf einen Hematokrit von 32% und eine Totalproteinkonzentration von 60 g/L eingestellt. Die Bestimmung des Hematokrit erfolgt mit einem Zellzählgerät (z.B. ABC Pentra 60, Axon Lab Ag) und die Bestimmung der Totalproteinkonzentration mit einem klinischen Analysator (z.B. Cobas c 111, Roche Diagnostics).

Der Membranfilter wird zunächst mit 1Liter Saline im single-pass und anschließend mit einem weiteren Liter Saline rezirkulierend (20 min, 200 ml/min) gespült. Im zweiten Spülschritt wird mittels einer externen Pumpe (MPC, Ismatec) Spülflüssigkeit über den Membranfilter in den Filtratraum abgezogen (60 ml/min). Die Saline wird dabei vollständig durch das Blut verdrängt und das Experiment bei 37°C mit Blutfluss $Q_B$=300 ml/min und einem Filtratfluss $Q_F$=60 ml/min (= 20% des Blutflusses) gestartet. Nach 60 min werden die Proben vom Blutein- und - ausgang sowie eine Filtratprobe genommen, daraus durch Zentrifugation Plasma gewonnen und die Konzentrationen von $\alpha_1$ saurem Glykoprotein, Albumin und IgG mittels Laser-Nephelometrie bestimmt (BN ProSpec, Siemens Diagnostics). Die Siebkoeffizientenberechnung erfolgt wie in Punkt 5.6.2.4 der DIN EN ISO 8637:2014-03 beschrieben.

Trenngrenze:

Zur Ermittlung der Trenngrenze werden die nach der zuvor beschriebenen Methode ermittelten Siebkoeffizienten für $\alpha_1$ saures Glykoprotein ($M_W$= 44.000 Dalton), $SK_{Gp}$, Albumin ($M_W$=68.000 Dalton), $SK_{Alb}$, und für Immunglobulin G ($M_W$=180.000 Dalton), $SK_{IgG}$, in ein Diagramm über dem Molekulargewicht aufgetragen. Unter Einbeziehung angenommener Eckpunkte bei 10.000 Dalton mit einem Siebkoeffizienten SK=1 und bei 1 Mio. Dalton mit einem Siebkoeffizienten SK=0 wird eine Ausgleichskurve durch die Punkte gelegt. Als Trenngrenze wird das Molekulargewicht bei einem Rückhalt von 95% bzw. bei einem Siebkoeffizienten SK von 0,05 ermittelt.

Ultrafiltrationsrate in Wasser $UFR_{Wasser}$ (hydraulische Permeabilität):

Hohlfasermembranen:

Zur Bestimmung der hydraulischen Permeabilität bzw. der Ultrafiltrationsrate in Wasser $UFR_{Wasser}$ der im Membranfilter enthaltenen semipermeablen Membran wird aus den zu prüfenden Hohlfasermembranen eine Prüfzelle mit definierter Hohlfaserzahl und Länge gefertigt. Die Hohlfasern werden dafür beidseitig an ihren Enden in Heißwachs eingebettet. Nach dem Aushärten des Wachses werden die Einbettungen freigeschnitten, damit die Lumina der Hohlfasermembranen durch den Schnitt geöffnet werden. Die Hohlfaserlumina in den Einbettungen müssen auf Durchgängigkeit überprüft werden. Die Länge der Prüfzelle beträgt üblicherweise 300 +/- 5 mm. Die Anzahl der Hohlfasermembranen liegt in der Regel zwischen 160 - 240.

Die effektive Fläche einer Prüfzelle definiert sich wie folgt:

$$A = n \cdot l \cdot d_i \cdot \pi \cdot f_{dim} \quad [m^2]$$

mit

A = effektive Fläche [$m^2$]
n = Anzahl der Kapillaren
l = freie Länge der Kapillaren [mm]
$d_i$ = Innendurchmesser der Kapillaren [$\mu$m]
$f_{dim}$ = Dimensionsfaktor [$1 \cdot 10^{-9}$ $m^2/(mm \cdot \mu m)$]

Die Prüfzelle wird vor der Messung für mindestens 15 Minuten bei Raumtemperatur in VE-Wasser gelagert (Benetzung) und danach in eine Prüfapparatur eingebunden. Die Messung wird mit auf 37°C temperiertem ultrafiltriertem und vollentsalztem Wasser durchgeführt. Die Prüfzelle ist während der Messung komplett in temperiertem Wasser getaucht. Der Prüfdruck vor der Prüfzelle wird auf 200$\pm$2 mbar eingestellt. Bei der Messung handelt es sich um eine Dead-End Methode. Die Prüfzelle wird zunächst für 900 s unter Prüfdruck konditioniert. Die eigentliche Messzeit im Anschluss beträgt 60 s, in der das während der Messung erzeugte Permeat volumetrisch erfasst wird.

Die $UFR_{Wasser}$ wird nach der folgenden Formel ermittelt:

$$UFR_{Wasser} = \frac{V_W}{\frac{\Delta t}{3600} \cdot A \cdot \left(\frac{P_O + P_E}{2}\right) \cdot f_{torr}} \quad [ml/(h\ m^2\ mmHg)]$$

Hierbei sind:

$V_W$ = durch die Membranprobe während der Messzeit hindurch geströmte Wasservolumen [ml]
$\Delta$t = Messzeit [s]
A = effektive Fläche [$m^2$]
$P_0$ = Prüfdruck [mbar] (Druck vor der Prüfzelle)
$P_E$ = Enddruck [mbar] (Druck nach der Prüfzelle)
$f_{torr}$ = 1/1,33322; Umrechnung [mbar] auf [mmHg]

Flachmembranen:

Aus der zu prüfenden Flachmembran werden scheibenförmige Membranproben mit einem Durchmesser von 15 cm ausgestanzt und in einen geeigneten Probenhalter am Umfang fluiddicht so eingespannt, dass eine freie Messfläche von 43,20 $cm^2$ resultiert. Der Probenhalter befindet sich in einem Gehäuse, das von Wasser druckbeaufschlagt durchströmt werden kann. Die eingespannte Membranprobe wird dann zunächst in auf 37°C temperiertem, vollentsalztem Wasser quellen lassen und anschließend von auf 37°C temperiertem, vollentsalztem Wasser unter einem definierten Druck zwischen 0,4 und 1,0 bar durchströmt. Nach einer Vorlaufzeit von 50 s bis zum Konstantwerden des Druckes wird das während einer Messzeit von 60 s durch die Membranprobe hindurch geströmte Wasservolumen gravimetrisch oder volumetrisch ermittelt.

Die Ultrafiltrationsrate $UFR_{Wasser}$ wird nach der Formel

$$UFR_{Wasser} = 800 \cdot \frac{V_W}{\Delta t \cdot A \cdot p_O} \cdot [ml/(h\ m^2\ mmHg)]$$

ermittelt. Hierbei sind:

$V_W$ = durch die Membranprobe während der Messzeit hindurch geströmte Wasservolumen [ml]
$\Delta t$ = Messzeit [min]
A = durchströmte Fläche der Membranprobe (43,20 $cm^2$)
$p_O$ = eingestellter Druck während der Messung [bar]

**Patentansprüche**

1. Blutbehandlungssystem zur Therapie akuter, durch Mediatoren vermittelte inflammatorischer Erkrankungen, umfassend mindestens eine erste Vorrichtung und mindestens eine zweite Vorrichtung,

   - wobei die mindestens eine erste Vorrichtung einen ersten blutseitigen Strömungspfad zum Hindurchleiten von Blut und die mindestens eine zweite Vorrichtung einen zweiten blutseitigen Strömungspfad zum Hindurchleiten von Blut aufweist und wobei die mindestens eine erste Vorrichtung und die mindestens eine zweite Vorrichtung so seriell hintereinandergeschaltet sind, dass der erste blutseitige Strömungspfad in Fluidverbindung mit dem zweiten blutseitigen Strömungspfad steht,
   - wobei die mindestens eine erste Vorrichtung ein Membranfilter zur Entfernung von toxischen Mediatoren aus Blut ist und die mindestens eine zweite Vorrichtung zur Entfernung von Granulozyten und Monozyten aus Blut geeignet ist und
   - wobei der Membranfilter ein Gehäuse, einen im Gehäuse ausgebildeten Filterinnenraum und eine im Filterinnenraum angeordnete semipermeable Membran aufweist, die den Filterinnenraum in einen Retentatraum und einen Permeatraum unterteilt,
   - wobei das Gehäuse eine Bluteinlasseinrichtung und eine Blutauslasseinrichtung aufweist, die mit dem Retentatraum in Fluidverbindung stehen, und die Bluteinlasseinrichtung, der Retentatraum und die Blutauslasseinrichtung den ersten blutseitigen Strömungspfad zum Hindurchleiten von Blut durch die erste Vorrichtung ausbilden und
   - wobei das Gehäuse des Weiteren einen Permeatauslass zum Ableiten von durch die semipermeable Membran hindurchtretendem Permeat aus dem Permeatraum aufweist,
   - wobei die semipermeable Membran eine Ultrafiltrationsrate in Wasser $UFR_{wasser}$ im Bereich von 500 bis 2000 ml/(h $m^2$ mmHg) und der Membranfilter eine Trenncharakteristik derart aufweist, dass der Siebkoeffizient für Albumin $SK_{Alb}$ im Bereich von 0,05 bis 0,3 und der Siebkoeffizient für Immunglobulin G (IgG), $SK_{IgG}$, im Bereich von 0,001 bis 0,1 liegt.

2. System nach Anspruch 1, **dadurch gekennzeichnet, dass** die semipermeable Membran mindestens eine semipermeable Hohlfasermembran mit einer Wand und einem von der Wand umschlossenen Lumen ist und dass der Retentatraum vom Lumen der mindestens einen Hohlfasermembran ausgebildet wird.

3. System nach Anspruch 1 oder 2, **dadurch gekennzeichnet, dass** die mindestens eine Hohlfasermembran über ihre Wand eine asymmetrische Porenstruktur mit einer Trennschicht auf der dem Lumen zugewandten Seite der

Wand der Hohlfasermembran aufweist.

4. System nach einem oder mehreren der Ansprüche 1 bis 3, **dadurch gekennzeichnet, dass** die zweite Vorrichtung ein Filter und/oder ein Adsorber ist.

5. System nach Anspruch 4, **dadurch gekennzeichnet, dass** die mindestens eine zweite Vorrichtung ein Filter zur Entfernung von Granulozyten und Monozyten ist

- mit einem Gehäuse, welches einen Innenraum sowie eine Einlasseinrichtung und eine Auslasseinrichtung aufweist, die mit dem Innenraum in Fluidverbindung stehen,
- wobei im Innenraum des Filtergehäuses ein Strömungskanäle aufweisendes und von Blut durchströmbares Filtermaterial angeordnet ist,
- wobei Einlasseinrichtung, Auslasseinrichtung und die Strömungskanäle des Filtermaterials im Innenraum den zweiten Strömungspfad ausbilden,
- und wobei das Filtermaterial für die Abtrennung von Granulozyten und Monozyten durch Größenausschluss und/oder Adsorption angepasst ist.

6. System nach Anspruch 4, **dadurch gekennzeichnet, dass** die mindestens eine zweite Vorrichtung zur Entfernung von Granulozyten und Monozyten aus Blut ein Adsorber mit einem Gehäuse ist, welches eine einen Innenraum umschließende Innenseite sowie eine Einlasseinrichtung und eine Auslasseinrichtung aufweist,

- wobei im Innenraum eine Vielzahl von Fäden angeordnet ist,
- wobei die Fäden mit zumindest einem ihrer Enden so in eine mit der Gehäuseinnenseite verbundenen Vergussmasse eingebettet sind, dass um die Fäden herum ein von Blut durchströmbarer Außenraum ausgebildet ist, der mit der Einlasseinrichtung und der Auslasseinrichtung in Fluidverbindung steht, wodurch Einlasseinrichtung, Auslasseinrichtung und Innenraum den zweiten Strömungspfad ausbilden,
- wobei die Anordnung der Fäden einen hohen Grad an Ordnung aufweist, wobei unter einem hohen Grad an Ordnung zu verstehen ist, dass ein Anteil von mindestens 25% der Fäden entlang ihrer Erstreckungsrichtung nebeneinander angeordnet sind und
- wobei die Fäden auf Basis organischer Polymere eine Bildung des Komplementaktivierungsproduktes C5a in einer Konzentration von mindestens 10 $\mu$g pro $m^2$ Fadenoberfläche bewirken.

7. System nach Anspruch 6, **dadurch gekennzeichnet, dass** die Fäden Hohlfäden sind mit einem Lumen und einer das Lumen umschließenden Wand sowie mit einer inneren, lumenseitigen Oberfläche und einer äußeren Oberfläche, wobei die Hohlfäden im Gehäuse so angeordnet sind, dass nur die äußere Oberflächen der Hohlfäden für das den Außenraum durchströmende Blut zugänglich, die Lumina der Hohlfäden hingegen nicht für ein Fluid zugänglich sind.

8. System nach Anspruch 4, **dadurch gekennzeichnet, dass** die mindestens eine zweite Vorrichtung zur Entfernung von Granulozyten und Monozyten aus Blut ein Adsorber mit einem Gehäuse ist, welches eine einen Innenraum umschließende Innenseite sowie eine Einlasseinrichtung und eine Auslasseinrichtung aufweist,

- wobei im Innenraum ein aus Partikeln aufgebautes Adsorptionsmaterial angeordnet ist und
- wobei um die Partikel des Adsorptionsmaterials herum ein von Blut durchströmbarer Außenraum ausgebildet ist, der mit der Einlasseinrichtung und der Auslasseinrichtung in Fluidverbindung steht, wodurch Einlasseinrichtung, Auslasseinrichtung und Innenraum den zweiten Strömungspfad ausbilden.

9. System nach einem oder mehreren der Ansprüche 1 bis 8, **dadurch gekennzeichnet, dass** eine erste Vorrichtung und eine zweite Vorrichtung die Gestalt von Zylindern haben, die direkt über eine Klebe-, Schweiß-, Schraub- oder Flanschverbindung miteinander verbunden sind.

## Claims

1. A blood treatment system for treating acute inflammatory diseases mediated by mediators, comprising at least one first device and at least one second device,

- wherein the at least one first device has a first blood-side flow path for conducting blood, and the at least one second device has a second blood-side flow path for conducting blood, and wherein the at least one first device

and the at least one second device are connected serially, one after the other, such that the first blood-side flow path is fluidically connected to the second blood-side flow path,
- wherein the at least one first device is a membrane filter for removing toxic mediators from blood, and the at least one second device is suitable for removing granulocytes and monocytes from blood; and
- wherein the membrane filter has a housing, a filter interior formed in the housing, and a semi-permeable membrane arranged in the filter interior, said membrane subdividing the filter interior into a retentate space and a permeate space,
- wherein the housing has a blood inlet device and a blood outlet device which are fluidically connected to the retentate space, and the blood inlet device, the retentate space, and the blood outlet device form the first blood-side flow path for conducting blood through the first device, and
- wherein the housing furthermore has a permeate outlet for discharging the permeate flowing through the semi-permeable membrane from the permeate space,
- wherein the semi-permeable membrane has an ultrafiltration rate in water UFRwater ranging from 500 to 2000 mL/(h $m^2$ mmHg), and the membrane filter has a separating characteristic such that the filter coefficient for albumin, $SK_{Alb}$, ranges from 0.05 to 0.3, and the filter coefficient for immunoglobulin G (IgG), $SK_{IgG}$, ranges from 0.001 to 0.1.

2. The system according to claim 1, **characterized in that** the semi-permeable membrane is at least one semi-permeable hollow fiber membrane having a wall and a lumen enclosed by the wall, and **in that** the retentate space is formed by the lumen of the at least one hollow fiber membrane.

3. The system according to either claim 1 or 2, **characterized in that** the at least one hollow fiber membrane has an asymmetrical pore structure across its wall, with a separating layer on the side of the wall of the hollow fiber membrane that faces toward the lumen.

4. The system according to one or more of claims 1 to 3, **characterized in that** the second device is a filter and/or an adsorber.

5. The system according to claim 4, **characterized in that** the at least one second device is a filter for removing granulocytes and monocytes

- with a housing which has an interior, as well as an inlet device and an outlet device which have a fluidic connection with the interior,
- wherein a filter material having flow channels and through which blood can flow is arranged in the interior of the filter housing,
- wherein inlet device, outlet device, and the flow channels of the filter material form the second flow path in the interior,
- and wherein the filter material is adapted for the separation of granulocytes and monocytes by means of size exclusion and/or adsorption.

6. The system according to claim 4, **characterized in that** the at least one second device for removing granulocytes and monocytes from blood is an adsorber with a housing which has an inner side surrounding the interior, as well as an inlet device and an outlet device,

- wherein a plurality of threads is arranged in the interior,
- wherein the threads are embedded with at least one of their ends in a casting compound connected to the housing inner side such that an exterior which allows the passage of blood is formed around the threads, with said exterior being in a fluidic connection with the inlet device and the outlet device, whereby the inlet device, the outlet device, and the interior form the second flow path,
- wherein the arrangement of the threads has a high level of organization, wherein the phrase "high level of organization" is understood to mean that a proportion of at least 25 % of the threads are arranged next to one another along their extension direction, and
- wherein the threads based on organic polymers produce a formation of the complement activation product C5a in a concentration of at least 10 $\mu$g per $m^2$ of thread surface.

7. The system according to claim 6, **characterized in that** the threads are hollow threads with a lumen and a wall surrounding the lumen, as well as with an inner, lumen-side surface and an outer surface, wherein the hollow threads are arranged in the housing such that only the outer surfaces of the hollow threads are accessible to the blood

flowing through the exterior, by contrast to which the lumina of the hollow threads are not accessible to a fluid.

8. The system according to claim 4, **characterized in that** the at least one second device for removing granulocytes and monocytes from blood is an adsorber with a housing which has an inner side surrounding the interior, as well as an inlet device and an outlet device,

- wherein adsorption material made up of particles is arranged in the interior, and
- wherein an exterior which blood can flow through is formed around the particles of the adsorption material, with the exterior being in a fluid connection with the inlet device and the outlet device, whereby the inlet device, the outlet device, and the interior form the second flow path.

9. The system according to one or more of claims 1 to 8, **characterized in that** a first device and a second device have the shape of cylinders which are directly connected to one another by means of an adhesive, welded, threaded, or flanged connection.

## Revendications

1. Système de traitement du sang pour la thérapie de maladies inflammatoires aigües, médiées par des médiateurs, comprenant au moins un premier dispositif et au moins un deuxième dispositif,

- ledit au moins un premier dispositif présentant une première voie d'écoulement côté sang pour le passage du sang et ledit au moins un deuxième dispositif présentant une deuxième voie d'écoulement côté sang pour le passage du sang et ledit au moins un premier dispositif et ledit au moins un deuxième dispositif étant commutés en série l'un derrière l'autre de manière telle que la première voie d'écoulement côté sang est en communication fluidique avec la deuxième voie d'écoulement côté sang,
- ledit au moins un premier dispositif étant un filtre à membrane pour l'élimination de médiateurs toxiques du sang et ledit au moins un deuxième dispositif étant approprié pour l'élimination de granulocytes et de monocytes du sang et
- le filtre à membrane présentant un boîtier, un espace interne de filtre formé dans le boîtier et une membrane semi-perméable, agencée dans l'espace interne de filtre, qui divise l'espace interne de filtre en un espace de rétentat et un espace de perméat,
- le boîtier présentant un dispositif d'entrée de sang et un dispositif de sortie du sang qui sont en communication fluidique avec l'espace de rétentat et le dispositif d'entrée de sang, l'espace de rétentat et le dispositif de sortie du sang formant la première voie d'écoulement côté sang pour le passage du sang à travers le premier dispositif et
- le boîtier présentant en outre une sortie de perméat pour l'évacuation du perméat traversant la membrane semi-perméable hors de l'espace de perméat,
- la membrane semi-perméable présentant une vitesse d'ultrafiltration dans l'eau $VUF_{eau}$ dans la plage de 500 à 2000 ml/(h $m^2$ mmHg) et le filtre à membrane présentant une caractéristique de séparation telle que le coefficient de filtration pour l'albumine $CF_{Alb}$ se situe dans la plage de 0,05 à 0,3 et le coefficient de filtration pour l'immunoglobuline G (IgG), $CF_{IgG}$, se situe dans la plage de 0,001 à 0,1.

2. Système selon la revendication 1, **caractérisé en ce que** la membrane semi-perméable est au moins une membrane semi-perméable à fibres creuses, pourvue d'une paroi et d'une lumière entourée par la paroi et **en ce que** l'espace de rétentat est formé par la lumière de ladite au moins une membrane à fibres creuses.

3. Système selon la revendication 1 ou 2, **caractérisé en ce que** ladite au moins une membrane à fibres creuses présente à travers sa paroi une structure asymétrique de pores pourvue d'une couche de séparation sur le côté orienté vers la lumière de la paroi de la membrane à fibres creuses.

4. Système selon l'une ou plusieurs des revendications 1 à 3, **caractérisé en ce que** le deuxième dispositif est un filtre et/ou un adsorbant.

5. Système selon la revendication 4, **caractérisé en ce que** ledit au moins un deuxième dispositif est un filtre pour l'élimination de granulocytes et de monocytes

- pourvu d'un boîtier qui présente un espace interne ainsi qu'un dispositif d'entrée et un dispositif de sortie, qui sont en communication fluidique avec l'espace interne,

- un matériau filtrant, présentant des canaux d'écoulement et pouvant être traversé par un flux de sang, étant agencé dans l'espace interne du boîtier de filtre,
- le dispositif d'entrée, le dispositif de sortie et les canaux d'écoulement du matériau filtrant formant, dans l'espace interne, la deuxième voie d'écoulement,
- et le matériau filtrant étant adapté à la séparation de granulocytes et de monocytes par exclusion de taille et/ou par adsorption.

**6.** Système selon la revendication 4, **caractérisé en ce que** ledit au moins un deuxième dispositif pour l'élimination de granulocytes et de monocytes du sang est un adsorbant pourvu d'un boîtier qui présente un côté interne entourant un espace interne ainsi qu'un dispositif d'entrée et un dispositif de sortie,

- une pluralité de fils étant agencés dans l'espace interne,
- les fils étant incorporés par l'intermédiaire d'au moins une de leurs extrémités dans une masse de coulée reliée au côté interne du boîtier de manière telle qu'un espace externe pouvant être traversé par un flux de sang est formé autour des fils, qui est en communication fluidique avec le dispositif d'entrée et le dispositif de sortie, moyennant quoi le dispositif d'entrée, le dispositif de sortie et l'espace interne forment la deuxième voie d'écoulement,
- l'agencement des fils présentant un degré d'ordre élevé, un degré d'ordre élevé signifiant qu'une proportion d'au moins 25 % des fils sont agencés les uns à côté des autres le long de leur direction d'extension et
- les fils à base de polymères organiques provoquant une formation du produit d'activation du complément C5a en une concentration d'au moins 10 $\mu$g par $m^2$ de surface des fils.

**7.** Système selon la revendication 6, **caractérisé en ce que** les fils sont des fils creux pourvus d'une lumière et d'une paroi entourant la lumière ainsi que d'une surface interne, côté lumière, et d'une surface externe, les fils creux étant agencés dans le boîtier de manière telle que seules les surfaces externes des fils creux sont accessibles pour le sang s'écoulant à travers l'espace externe, les lumières des fils creux n'étant par contre pas accessibles pour un fluide.

**8.** Système selon la revendication 4, **caractérisé en ce que** ledit au moins un deuxième dispositif pour l'élimination de granulocytes et de monocytes du sang est un adsorbant pourvu d'un boîtier qui présente un côté interne entourant un espace interne ainsi qu'un dispositif d'entrée et un dispositif de sortie,

- un matériau d'adsorption constitué par des particules étant agencé dans l'espace interne et
- un espace externe, en communication fluidique avec le dispositif d'entrée et le dispositif de sortie, pouvant être traversé par un flux de sang, étant formé autour des particules du matériau d'adsorption, suite à quoi le dispositif d'entrée, le dispositif de sortie et l'espace interne forment la deuxième voie d'écoulement.

**9.** Système selon l'une ou plusieurs des revendications 1 à 8, **caractérisé en ce qu'**un premier dispositif et un deuxième dispositif présentent la forme de cylindres qui sont reliés directement l'un à l'autre par une liaison collée, soudée, vissée ou à bride.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5571418 A
- US 20120312732 A
- EP 2281625 A
- WO 03009885 A
- WO 2011131534 A
- DE 19913707 A
- WO 2013025483 A
- WO 2012094565 A
- US 20130011824 A
- WO 0002603 A
- EP 0787500 A
- EP 0958839 A
- US 20130161247 A1
- WO 2007025735 A
- EP 0155003 A
- EP 1444996 A
- EP 1553113 A
- EP 1582228 A
- EP 1754496 A
- US 20110031191 A
- WO 2004018078 A
- WO 2004039474 A
- WO 2005002647 A
- WO 2006061862 A
- EP 0478914 A
- EP 0606646 A
- EP 1016426 A
- US 4476023 A
- WO 2004064980 A
- WO 2008028807 A
- EP 1666129 A
- US 5478470 A
- US 20080203024 A
- US 20100084331 A
- EP 0319961 A
- EP 1882738 A
- WO 200055621 A
- US 4370381 A